# FASCICULE DE BREVET EUROPEEN

(11) **EP 1 356 799 B1**
(45) Date de publication et mention de la délivrance du brevet: **30.05.2018**
(21) Numéro de dépôt: 02291066.5
(22) Date de dépôt: 26.04.2002
(51) Int. Cl.: A61Q 1/10, A61K 8/81, A61K 8/87

(54) **Composition cosmétique filmogène**
Filmbildende kosmetische Zusammensetzung
Film-forming cosmetic composition

(30) Priorité: 04.05.2001 FR 0106042
(43) Date de publication de la demande: 29.10.2003
(73) Titulaire: L'Oréal, 75008 Paris (FR)
(72) Inventeur: De la Poterie, Valérie, 77820 Le Châtelet en Brie (FR)
(74) Mandataire: Kromer, Christophe

(56) Documents cités:
- EP-A- 0 367 015
- EP-A- 0 847 753
- EP-A- 1 010 418
- EP-A- 1 163 896
- EP-A- 1 281 385
- WO-A-92/07913
- WO-A-95/15741
- WO-A-96/36308
- US-A- 4 423 031
- US-A- 5 156 911
- US-A- 5 874 072
- US-A- 5 985 258
- PATENT ABSTRACTS OF JAPAN vol. 2000, no. 11, 3 janvier 2001 (2001-01-03) & JP 2000 219618 A (SHISEIDO CO LTD), 8 août 2000 (2000-08-08)

## Description

La présente invention concerne une composition cosmétique comprenant un polymère semi-cristallin et un polymère apte à former un film hydrophobe, utilisable comme composition de maquillage ou de soin des matières kératiniques comme la peau, les cils, les sourcils, les cheveux et les ongles, notamment d'êtres humains

L'invention a aussi pour objet un procédé de maquillage ou de soin cosmétique des matières kératiniques.

La composition peut se présenter sous la forme de mascara, d'eye-liner, de produit pour les lèvres, de fard à joues ou à paupières, de fond de teint, de produit de maquillage du corps, de produit anti-cernes, de produit pour les ongles, de composition de protection solaire, de coloration de la peau, de produit de soin de la peau. Plus spécialement, l'invention porte sur un mascara.

Il est connu du document WO-A-95/15741 des compositions de mascaras sous forme d'émulsion cire-dans-eau comprenant des tensio-actifs. Toutefois, le film de maquillage obtenu avec ces compositions ne présente pas une bonne résistance à l'eau et le film au contact de l'eau, pendant la baignade ou la douche par exemple, se désagrège en partie en s'effritant ou bien encore en s'étalant autour de l'oeil. L'effritement du film engendre une perte sensible de l'intensité de la couleur du maquillage, obligeant ainsi la consommatrice à renouveler l'application du mascara. L'étalement du film forme, quant à lui, une auréole autour de la zone maquillée très inesthétique. Les larmes et la transpiration provoquent également ces mêmes inconvénients.

Pour favoriser la tenue à l'eau du maquillage, il est connu du document US-A-4423031 d'utiliser des polymères acryliques en dispersion aqueuse. Néanmoins, le mascara est difficile à démaquiller et nécessite l'emploi de démaquillants spécifiques à base d'huiles ou de solvants organiques. Or ces démaquillants peuvent être irritants pour l'oeil, notamment provoquer des picotements ou laisser un voile sur l'oeil, ou bien encore peuvent laisser sur la peau autour de l'oeil (paupières) un film résiduel gras inconfortable.

Pour éviter l'emploi de ces démaquillants spécifiques, il est possible d'employer de l'eau et du savon comme le décrit le document WO-A-96/33690 en proposant un mascara comprenant un polymère insoluble dans l'eau et un polymère filmogène hydrosoluble. Toutefois, l'emploi de savon peut provoquer un inconfort occulaire dû à des picotements ou au dépôt d'un voile sur l'oeil. Le savon solubilise aussi le film de maquillage qui s'étale alors autour de l'oeil et forme des auréoles inesthétiques et tache la peau.

Le document EP1 010 418 propose d'utiliser des polyuréthanes filmogènes pour obtenir des mascaras de bonne tenue. Le document EP 1 163 896 divulgue des mascaras ayant une bonne tenue à l'eau comprenant un polymère filmogène hydrophobe et un agent de transition thermique. Le document EP 0 847 753 décrit des compositions sans transfert comprenant un polymère filmogène et une dispersion aqueuse de cires.

US5318995 divulgue des compositions comprenant un polymère d'alkyl acrylamide avec un groupe alkyle en C12 à C18. L'emploi d'eau chaude, c'est-à-dire d'eau ayant une température supérieure ou égale à 35 °C (température mesurée à la pression atmosphérique), et notamment allant environ de 35 °C à 50 °C, permet d'éviter les inconvénients des démaquillants connus jusqu'à présents mais les compositions de mascara résistante à l'eau froide décrites précédemment ne sont pas éliminables à l'eau chaude.

Le but de la présente invention est donc de proposer une composition cosmétique démaquillable à l'eau chaude tout en présentant une bonne tenue à l'eau froide.

Les inventeurs ont découvert qu'une telle composition pouvait être obtenue en utilisant un polymère filmogène apte à former un film hydrophobe et un polymère semi-cristallin particulier. Après application de la composition sur les matières kératiniques, notament sur les cils, le maquillage obtenu est bien résistant à l'eau froide, c'est-à-dire à une eau ayant une température inférieure ou égale à 30 °C, lors de baignade par exemple, et/ou aux larmes et/ou à la transpiration. Le maquillage s'élimine facilement avec de l'eau chaude, notamment par frottements avec un coton ou une gaze : le maquillage se décolle facilement des cils et est retiré des cils sans se fragmenter (en forme de gaine) ou sous forme de fragments ou de morceaux. Le maquillage ainsi éliminé ne s'étale pas sur la peau ce qui évite la formation d'auréoles autour de l'oeil ; la peau n'est pas tachée lors du démaquillage et reste propre. Le maquillage s'élimine très simplement avec de l'eau chaude et en particulier avec de l'eau chaude ne contenant pas d'agent détergent tel que les savons. Pour le démaquillage, l'eau chaude utilisée peut être de l'eau de robinet, de l'eau déminéralisée ou bien encore de l'eau minérale portées à une température supérieure ou égale à 35 °C, et notamment allant environ de 35 °C à 50 °C.

De façon plus précise, l'invention a pour objet une composition comprenant, dans un milieu physiologiquement acceptable, au moins un premier polymère semi-cristallin ayant un point de fusion supérieur ou égal à 30 °C, et au moins un deuxième polymère filmogène apte à former un film hydrophobe à température ambiante tels que définis en revendication 1.

L'invention a aussi pour objet l'utilisation d'une composition telle que définie précédement pour obtenir un film déposé sur les matières kératiniques résistant à l'eau froide et/ou démaquillable à l'eau chaude.

L'invention a encore pour objet un procédé cosmétique de maquillage ou de soin des matières kératiniques comprenant l'application sur les matières kératiniques d'une composition telle que définie précédemment.

L'invention a également pour objet l'utilisation d'un premier polymère semi-cristallin ayant un point de fusion supérieur ou égal à 30 °C, et au moins un deuxième polymère filmogène apte à former un film hydrophobe à température ambiante, dans une composition cosmétique de maquillage ou de soin des matières kératiniques, pour obtenir un film déposé sur les matières kératiniques résistant à l'eau froide et/ou démaquillable à l'eau chaude.

L'invention a aussi pour objet un procédé cosmétique de démaquillage des matières kératiniques maquillées avec une composition telle que définie précédemment, comprenant au moins une étape de rinçage avec de l'eau chaude portée à une température supérieure ou égale à 35 °C des dites matières kératiniques maquillées.

Par physiologiquement acceptable, il faut comprendre un milieu compatible avec les matières kératiniques, comme un milieu cosmétique.

Avantageusement, la composition selon l'invention contient peu, voire est exempte, d'agent émulsionnant (tensio-actif), notamment en une teneur inférieure à 0,5 % en poids, par rapport au poids total de la composition. La composition présente ainsi une bonne tenue à l'eau froide.
On entend par émulsionnant tout composé amphiphile choisi parmi les composés amphiphiles non-ioniques ayant un HLB (hydrophile-lipophile balance) supérieur ou égale à 10 et les composés amphiphiles ioniques dont la partie hydrophile comprend un contre-ion ayant une masse molaire supérieure ou égale à 50 g/mole.

Le démaquillage à l'eau chaude est obtenu en utilisant un polymère semi-cristallin présentant une transition thermique correspondant à sa température de fusion.
Au dessus de sa température de fusion, le polymère semi-cristallin après son changement d'état rend le film plus sensible à l'eau : le film de maquillage est fragilisé lors du contact avec l'eau chaude et en le frottant, par exemple avec les doigts ou bien avec un tissu ou un coton, le film se désagrège facilement ou se décolle de son support.

Le polymère semi-cristallin est présent dans la composition en une teneur allant de 1 % à 30 % en poids, par rapport au poids total de la composition.

Par "polymères", on entend au sens de l'invention des composés comportant au moins 2 motifs de répétition, de préférence au moins 3 motifs de répétition et plus spécialement au moins 10 motifs de répétition.

Par "polymère semi-cristallin", on entend au sens de l'invention, des polymères comportant une partie cristallisable, chaîne pendante cristallisable ou séquence cristallisable dans le squelette, et une partie amorphe dans le squelette et présentant une température de changement de phase réversible du premier ordre, en particulier de fusion (transition solide-liquide). Lorsque la partie cristallisable est sous forme d'une séquence cristallisable du squelette polymérique, la partie amorphe du polymère est sous forme de séquence amorphe ; le polymère semi-cristallin est dans ce cas un copolymère séquencé par exemple du type dibloc, tri-bloc ou multibloc, comportant au moins une séquence cristallisable et au moins une séquence amorphe. Par "séquence", on entend généralement au moins 5 motifs de répétition identiques. La ou les séquences cristallisables sont alors de nature chimique différente de la ou des séquences amorphes.

Le polymère semi-cristallin selon l'invention a une température de fusion supérieure ou égale à 30 °C (notamment allant de 30 °C à 80 °C), de préférence allant de 30 °C à 60 °C. Cette température de fusion est une température de changement d'état du premier ordre.
Cette température de fusion peut être mesurée par toute méthode connue et en particulier à l'aide d'un calorimètre à balayage différentiel (D.S.C).

De façon avantageuse, le ou les polymères semi-cristallins auxquelles s'applique l'invention présentent une masse moléculaire moyenne en nombre supérieure ou égale à 1 000.

De façon avantageuse, le ou les polymères semi-cristallins de la composition de l'invention ont une masse moléculaire moyenne en nombre Mn allant de 2 000 à 800 000, de préférence de 3 000 à 500 000 , mieux de 4 000 à 150 000, notamment inférieure à 100 000, et mieux de 4 000 à 99 000. De préférence, ils présentent une masse moléculaire moyenne en nombre supérieure à 5 600, allant par exemple de 5 700 à 99 000.

Par "chaîne ou séquence cristallisable", on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitif du polymère. Avantageusement, la "chaîne pendante cristallisable" peut être chaîne comportant au moins 6 atomes de carbone.

De préférence, la ou les séquences ou chaînes cristallisables des polymères semi-cristallins représentent au moins 30 % du poids total de chaque polymère et mieux au moins 40 %. Les polymères semi-cristallins de l'invention à séquences cristallisables sont des polymères, séquencés ou multiséquencés. Ils peuvent être obtenus par polymérisation de monomère à double liaisons réactives (ou éthyléniques) ou par polycondensation. Lorsque les polymères de l'invention sont des polymères à chaînes latérales cristallisables, ces derniers sont avantageusement sous forme aléatoire ou statistique.

De préférence, les polymères semi-cristallins de l'invention sont d'origine synthétique. En outre, ils ne comportent pas de squelette polysaccharidique. De façon générale, les motifs (chaînes ou séquences) cristallisables des polymères semi-cristallins selon l'invention, proviennent de monomère(s) à séquence(s) ou chaîne(s) cristallisable(s), utilisé(s) pour la fabrication des polymères semi-cristallins.
Selon l'invention, le polymère semi-cristallin à bas point de fusion et le polymère semi-cristallin à haut point de fusion sont choisis parmi les copolymères séquencés comportant au moins une séquence cristallisable et au moins une séquence amorphe, les homopolymères et les copolymères portant au moins une chaîne latérale cristallisable par motif de répétition, leurs mélanges.

Les polymères semi-cristallins utilisables dans l'invention sont en particulier :
- les copolymères séquencés de polyoléfines à cristallisation contrôlée, notamment ceux dont les monomères sont décrits dans EP-A-0 951 897.
- les polycondensats et notamment de type polyester, aliphatique ou aromatique ou copolyester aliphatique/aromatique,
- les homo- ou co-polymères portant au moins une chaîne latérale cristallisable et les homo- ou co-polymères portant dans le squelette au moins une séquence cristallisable, comme ceux décrits dans le document US-A-5 156 911,
- les homo- ou co-polymères portant au moins une chaîne latérale cristallisable en particulier à groupement(s) fluoré(s) tels que décrits dans le document WO-A-01/19333,
et leurs mélanges. Dans ces deux derniers cas, la ou les chaînes latérales ou séquences cristallisables sont hydrophobes.

### Polymères semi-cristallins à chaînes latérales cristallisables

On peut citer en particulier ceux définis dans le document US-A-5156911 et WO-A-01/19333. Ce sont des homopolymères ou copolymères comportant de 50 à 100 % en poids de motifs résultant de la polymérisation d'un ou plusieurs monomères porteurs de chaîne latérale hydrophobe cristallisable.
Ces homo- ou co-polymères sont de toute nature du moment qu'ils présentent les conditions indiquées précédemment.
Ils peuvent résulter :
- de la polymérisation notamment radicalaire d'un ou plusieurs monomères à double(s) liaison(s) réactive(s) ou éthyléniques vis-à-vis d'une polymérisation, à savoir à groupe vinylique, (méth)acrylique ou allylique.
- de la polycondensation d'un ou plusieurs monomères porteurs de groupes co-réactifs (acide carboxylique ou sulfonique, alcool, amine ou isocyanate), comme par exemple les polyesters, les polyuréthanes, les polyéthers, les polyurées, les polyamides.

Lorsque les polymères résultent d'une polycondensation, les chaînes cristallisables hydrocarbonées et/ou fluorées telles que définies ci-dessus, sont portées par un monomère qui peut être un diacide, un diol, une diamine, un di-isocyanate. Lorsque les polymères objets de l'invention sont des copolymères, ils contiennent, en plus, de 0 à 50 % de groupes Y ou Z résultant de la copolymérisation : Les polymères semi-cristallins à chaîne latérale cristallisable sont des homopolymères d'alkyl(méth)acrylate ou d'alkyl(méth)acrylamide avec un groupe alkyle tel que défini ci-dessus, et notamment en C₁₄-C₂₄, des copolymères de ces monomères avec un monomère hydrophile de préférence de nature différente de l'acide (méth)acrylique comme la N-vinylpyrrolidone ou l'hydroxyéthyl (méth)acrylate et leurs mélanges.

Les polymères semi-cristallins de la composition de l'invention peuvent être ou non réticulés en partie du moment que le taux de réticulation ne gène pas leur dissolution ou dispersion dans la phase grasse liquide par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère comme par exemple les interactions dipolaires entre ionomères carboxylates, ces interactions étant en faible quantité et portées par le squelette du polymère ; soit à une séparation de phase entre les séquences cristallisables et les séquences amorphes, portées par le polymère.
De préférence, les polymères semi-cristallins de la composition selon l'invention sont non réticulés.
Selon un mode particulier de réalisation de l'invention, le polymère est choisi parmi les copolymères résultant de la polymérisation d'au moins un monomère à chaîne cristallisable choisi parmi les (méth)acrylates d'alkyle saturés en C₁₄ à C₂₄, les N alkyl (méth)acrylamides en C₁₄ à C₂₄ qui peut être représenté par la formule suivante : 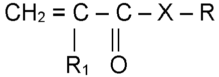 dans laquelle R₁ est H ou CH₃, R représente un groupe alkyle en C₁-C₁₀ éventuellement fluoré et X représente O, NH ou NR₂, où R₂ représente un groupe alkyle en C₁-C₁₀ éventuellement fluoré.
Selon un mode plus particulier de réalisation de l'invention, le polymère est issu d'un monomère à chaîne cristallisable choisi parmi les (méth)acrylates d'alkyle saturés en C₁₄ à C₂₂.

A titre d'exemple particulier de polymère semi-cristallin structurant utilisable dans la composition selon l'invention, on peut citer les produits Intelimer® de la société Landec décrits dans la brochure "Intelimer® polymers", Landec IP22 (Rev. 4-97). Ces polymères sont sous forme solide à température ambiante (25°C). Ils sont porteurs de chaînes latérales cristallisables et présentent la formule X précédente.
Les polymères semi-cristallins peuvent être notamment :
ceux décrits dans les exemples 3, 4, 5, 7, 9, 13 du brevet US-A-5 156 911 à groupement -COOH, résultant de la copolymérisation d'acide acrylique et d'alkyl(méth)acrylate en C₅ à C₁₆ et plus particulièrement de la copolymérisation :
• d'acide acrylique, d'hexadécylacrylate et d'isodécylacrylate dans un rapport pondéral 1/16/3,
• d'acide acrylique et de pentadécylacrylate dans un rapport pondéral 1/19,
• d'acide acrylique, d'hexadécylacrylate, éthylacrylate dans un rapport pondéral 2, 5/76, 5/20,
• d'acide acrylique, d'hexadécylacrylate et de méthylacrylate dans un rapport pondéral 5/85/10,
• d'acide acrylique et de octadécylméthacrylate dans un rapport pondéral 2,5/97,5,
• d'hexadécylacrylate, de monométhyl éther de méthacrylate polyéthylèneglycol à 8 motifs d'éthylèneglycol, et d'acide acrylique dans un rapport pondéral 8,5/1/0,5.

On peut aussi utiliser le structure « O » de National Starch tel que celui décrit dans le document US-A-5 736 125 de point de fusion 44°C ainsi que les polymères semi-cristallins à chaînes pendantes cristalisables comportant des groupements fluorés tels que décrits dans les exemples 1, 4, 6, 7 et 8 du document WO-A-01/19333.

On peut encore utiliser les polymères semi-cristallins de bas point de fusion obtenus par copolymérisation d'acrylate de stéaryle et d'acide acrylique ou de NVP tels que décrits dans le document US-A-5 519 063 ou EP-A-550745 et plus spécialement ceux décrits dans les exemples 1 et 2, ci-après, de préparation de polymère, de température de fusion respectivement de 40°C et 38°C.

On peut aussi utiliser les polymères semi-cristallins obtenus par copolymérisation de l'acrylate de béhényle et de l'acide acrylique ou de NVP tels que décrits dans les documents US-A-5519063 et EP-A-550745 et plus spécialement ceux décrits dans les exemples 3 et 4, ci-après, de préparation de polymère, de température de fusion respectivement de 60°C et 58°C.

De préférence, les polymères semi-cristallins à bas point de fusion et/ou ceux à haut point de fusion ne comportent pas de groupement carboxylique.

Selon l'invention, la composition comprend également un polymère filmogène apte à former un film hydrophobe, appelé aussi deuxième polymère filmogène.

Dans la présente demande, on entend par "polymère filmogène apte à former un film à température ambiante" un polymère apte à former, à 25 °C, à lui seul ou en présence d'agent auxiliaire de filmification, un film continu et adhérent sur un support, notamment sur les matières kératiniques.

Par "polymère filmogène apte à former un film hydrophobe", on entend un polymère dont le film a une solubilité dans l'eau à 25 °C inférieure à 1 % en poids.

Le deuxième polymère filmogène peut être choisi parmi les polymères synthétiques, notamment les polymères radicalaires ou les polycondensats, et leurs mélanges.

Par polymère filmogène radicalaire, on entend un polymère obtenu par polymérisation de monomères à insaturation notamment éthylénique (à l'inverse des polycondensats).

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

On peut ainsi citer, parmi les polycondensats utilisables comme polymère filmogène, les polyuréthannes anioniques, cationiques, non-ioniques ou amphotères, les polyuréthannes-acryliques, les polyuréthannes-polyvinylpirrolidones, les polyester-polyuréthannes, les polyéther-polyuréthannes, les polyurées, les polyurée/polyuréthannes, et leurs mélanges.
Le polyuréthanne filmogène peut être, par exemple, un copolymère polyuréthanne, polyurée/uréthanne, ou polyurée, aliphatique, cycloaliphatique ou aromatique, comportant seule ou en mélange :
- au moins une séquence d'origine polyester aliphatique et/ou cycloaliphatique et/ou aromatique, et/ou,
- au moins une séquence siliconée, ramifiée ou non, par exemple polydiméthylsiloxane ou polyméthylphénylsiloxane, et/ou
- au moins une séquence comportant des groupes fluorés. Le deuxième polymère est présent sous forme de particules solides dispersées dans un milieu aqueux. Par polymère sous forme de particules en dispersion aqueuse, connu généralement sous le nom de latex ou pseudolatex, on entend une phase contenant de l'eau et éventuellement un composé soluble dans l'eau, dans laquelle est dispersé directement le polymère sous forme de particules.
La taille des particules de polymères en dispersion aqueuse peut aller de 10 nm à 500 nm, et de préférence de 20 nm à 300 nm.
Le milieu aqueux peut être constitué essentiellement d'eau ou bien encore comprendre également un mélange d'eau et de solvant miscible à l'eau comme les monoalcools inférieurs ayant de 1 à 5 atomes de carbone, les glycols ayant de 2 à 8 atomes de carbone, les cétones en C₃-C₄, les aldéhydes en C₂-C₄. Il représente, en pratique, de 5 % à 94,9 % en poids, par rapport au poids total de la composition.
Comme polymère filmogène en dispersion aqueuse, on peut utiliser les polyuréthanes tels que les polyester-polyuréthanes vendus sous les dénominations "AVALURE UR-405®", "AVALURE UR-410®", "AVALURE UR-425®", "SANCURE 2060®" par la société GOODRICH, les polyéther-polyuréthanes vendus sous les dénominations "SANCURE 878®", "AVALURE UR-450®" par la société GOODRICH, "NEOREZ R 970®" par la société ICI, les polyuréthanes-acrylique vendus sous la dénomination NEOREZ R-989® par la société AVECIA-NEORESINS.

Il est également possible d'utiliser des polymères dits alcali-solubles en veillant à ce que le pH de la composition soit ajusté pour maintenir ces polymères à l'état de particules en dispersion aqueuse.

La composition selon l'invention peut comprendre un agent auxiliaire de filmification favorisant la formation d'un film avec les particules du polymère filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

De préférence, la phase grasse liquide comprend une phase grasse liquide volatile, éventuellement en mélange avec une phase grasse liquide non volatile.

Par "phase grasse volatile", on entend tout milieu non aqueux susceptible de s'évaporer de la peau en moins d'une heure. Cette phase volatile comporte notamment des huiles ayant une pression de vapeur, à température ambiante et pression atmosphérique allant de 10⁻³ à 300 mm de Hg (0,13 Pa à 40 000 Pa).

La phase grasse liquide dans laquelle est dispersé le polymère, peut être constituée de toute huile physiologiquement acceptable et en particulier cosmétiquement acceptable, notamment choisie parmi les huiles d'origine minérale, animale, végétale ou synthétique, carbonées, hydrocarbonées, fluorées et/ou siliconées, seules ou en mélange dans la mesure où elles forment un mélange homogène et stable et où elles sont compatibles avec l'utilisation envisagée.

La phase grasse liquide totale de la composition peut représenter de 5 % à 98 % en poids, par rapport au poids total de la composition et de préférence de 20 à 85 % en poids. La partie non volatile peut représenter de 0 à 80 % (notamment de 0,1 à 80 %) du poids total de la composition et mieux de 1 à 50%.

Comme phase grasse liquide utilisable dans l'invention, on peut ainsi citer les esters d'acide gras, les acides gras supérieurs, les alcools gras supérieurs, les poly-diméthylsiloxane (PDMS), éventuellement phénylées telles que les phényltriméthicones, ou éventuellement substitués par des groupements aliphatiques et/ou aromatiques, éventuellement fluorés, ou par des groupements fonctionnels tels que des groupements hydroxyle, thiol et/ou amine ; les polysiloxanes modifiés par des acides gras, des alcools gras ou des polyoxyalkylènes, les silicones fluorées, les huiles perfluorées.

Avantageusement, on peut utiliser une ou plusieurs huiles volatiles à température ambiante. Après évaporation de ces huiles, on obtient un dépôt filmogène souple, non collant. Ces huiles volatiles facilitent, en outre, l'application de la composition sur les fibres kératiniques comme les cils.

Ces huiles volatiles peuvent être des huiles hydrocarbonées, des huiles siliconées comportant éventuellement des groupements alkyle ou alkoxy en bout de chaîne siliconée ou pendante.

Comme huile de silicone volatile utilisable dans l'invention, on peut citer les silicones linéaires ou cycliques ayant de 2 à 7 atomes de silicium, ces silicones comportant éventuellement des groupes alkyle ou alkoxy ayant de 1 à 10 atomes de carbone. on peut citer notamment l'octaméthylcyclotétrasiloxane, le décaméthyl-cyclopentasiloxane, l'hexadécaméthylcyclohexasiloxane, l'heptaméthylhexyltrisiloxane, l'heptaméthyloctyltrisiloxane.

Comme huile hydrocarbonée volatile, on peut citer les isoparaffines en C₈-C₁₆ telles que les 'ISOPARs', les PERMETYLs et notamment l'isododécane.

Ces huiles volatiles peuvent être présentes dans la composition en une teneur allant de 5 à 94,9 % du poids total de la composition, et mieux de 20 à 85 %.

Le deuxième polymère filmogène peut être présent en une teneur en matière sèche allant de 5 % à 60 % en poids, par rapport au poids total de la composition, de préférence de 10 % à 45 % en poids, et mieux de 15 % à 35 % en poids.

Avantageusement, le premier polymère semi-cristallin et le deuxième polymère filmogène peuvent être présents dans la composition selon un rapport pondéral deuxième polymère filmogène / premier polymère semi-cristallin allant de 90/10 à 10/90, de préférence de 70/30 à 30/70, et mieux de 60/40 à 40/60.

La composition peut également comprendre au moins une matière colorante comme les composés pulvérulents et/ou les colorants liposolubles, par exemple à raison de 0,01 à 50 % du poids total de la composition. Les composés pulvérulents peuvent être choisis parmi les pigments et/ou les nacres habituellement utilisés dans les compositions cosmétiques. Avantageusement, les composés pulvérulents représentent de 0,1 à 25 % du poids total de la composition et mieux de 1 à20%.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.
La composition peut également comprendre des charges qui peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de Nylon (Orgasol de chez Atochem), de poly-β-alanine et de polyéthylène, le Téflon, la lauroyl-lysine, l'amidon, le nitrure de bore, les poudres de polymères de tétrafluoroéthylène, les microsphères creuses telles que l'Expancel (Nobel Industrie), le polytrap (Dow Corning) et les microbilles de résine de silicone (Tospearls de Toshiba, par exemple), le carbonate de calcium précipité, le carbonate et l'hydro-carbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.
La composition peut contenir, en outre, tout additif usuellement utilisés dans de telles compositions tels que les épaississants, les conservateurs, les parfums, les filtres solaires, les agents antiradicaux-libres, les cires, les huiles, les agents hydratants, les vitamines, les protéines, les plastifiants, les sequestrants, les céramides, les agents alcalinisants ou acidifiants, les émollients.

L'invention est illustrée plus en détail dans les exemples suivants.

### Test de mesure de la résistance à l'eau d'un film :

On étale une couche de composition de 300 µm d'épaisseur (avant séchage) d'une surface de 9 cm X 9 cm sur une plaque de verre d'une surface de 10 cm X 10 cm, on laisse sécher pendant 24 heures à 30 °C et 50 % d'humidité relative. Après séchage, on place la plaque dans un cristallisoir d'un diamètre de 19 cm et d'une contenance de 2 litres rempli d'un litre d'eau posé sur un agitateur magnétique chauffant vendu sous la dénomination RCT basic par la société IKA labor technik.

Puis on place sur le film un barreau aimanté cylindrique lisse en PTFE (longueur 6 cm ; diamètre 1 cm). On règle la vitesse d'agitation en position 5. La température de l'eau est controlée à l'aide d'un thermomètre à la température de 20 °C ou de 40 °C. Au temps to = 0, on débute l'agitation. On mesure le temps t (exprimé en minutes) au bout duquel le film commence à se détacher ou se décoller de la plaque ou lorsque l'on observe un trou de la taille du barreau magnétique d'agitation, c'est-à-dire lorsque le trou a un diamètre de 6 cm. Le test est arrêté si au bout de 2 heures le film reste intact. La résistance à l'eau du film correspond au temps t mesuré exprimé en minute.

### Test de reprise en eau d'un film :

On étale une couche de composition de 300 µm d'épaisseur (avant séchage) déposée sur une plaque puis séchée pendant 24 heures à 30 °C et à 50 % d'humidité relative ; des morceaux d'environ 1 cm² découpés dans le film sec sont pesés (mesure de la masse M1) puis immergés dans l'eau, à 20 °C ou à 40 °C, pendant 10 minutes ; après immersion, le morceau de film est essuyé pour éliminer l'excédent d'eau en surface puis pesé (mesure de la masse M2). La différence M2 - M1 correspond à la quantité d'eau absorbée par le film.
La reprise en eau du film est égale à [(M2 - M1) / M1] x 100 et est exprimée en pourcentage de poids d'eau par rapport au poids du film.

### Exemples de fabrication de polymères semi-cristallins

### Exemple 1 : Polymère acide de point de fusion de 40°C

Dans un réacteur d'1*l* muni d'une agitation centrale avec ancre, d'un réfrigérant et d'un thermomètre, on introduit 120g de Parléam que l'on chauffe de la température ambiante à 80°C en 45 min. A 80°C, on introduit en 2h le mélange C₁ suivant :
40g de cyclohexane + 4g de Triganox 141 [2,5 bis (2-éthyl hexanoyl peroxy) - 2,5 - diméthyl hexane].

30 min après le début de la coulée du mélange C₁, on introduit en 1h30 le mélange C₂ constitué de :
190g d'acrylate de stéaryle + 10g d'acide acrylique + 400 g de cyclohexane.
A la fin des deux coulées, on laisse agir 3h supplémentaires à 80°C puis on distille à pression atmosphérique la totalité du cyclohexane présent dans le milieu réactionnel.
On obtient alors le polymère à 60 % en poids en matière active dans le Parléam. Sa masse moléculaire moyenne en poids M_{w} est de 35 000 exprimée en équivalent polystyrène et sa température de fusion T_{f} est de 40°C ± 1°C, mesurée par D.S.C.

### Exemple 2 : Polymère basique de point de fusion de 38°C

On applique le même mode opératoire que dans l'exemple 1, sauf que l'on utilise de la N-Vinyl Pyrrolidone en lieu et place de l'acide acrylique.
Le polymère obtenu est à 60% en poids en matière active dans le Parléam, sa masse moléculaire moyenne en poids M_{w} est de 38 000 et sa T_{f} de 38°C.

### Exemples 3 et 4 :

On a testé les polymères semi-cristallins des exemples 1 et 2 dans la composition suivante :

| | | |
|---|---|---|
| - Polyuréthane en dispersion aqueuse vendu sous la dénomination AVALURE UR 425 par la société GOODRICH à 49 % en poids de matières actives | | 14 g MA |
| - Polymère semi-cristallin | | 10 g MA |
| - Hydroxyéthylcellulose | | 1,9 g |
| - Oxyde de fer noir | | 5 g |
| - Propylène glycol | | 5 g |
| - Conservateurs | qs | |
| - Eau | qsp | 100 g |

Pour chaque composition, on a mesuré la résistance (notée RES) à l'eau froide (20 °C) et à l'eau chaude (40 °C), et la reprise en eau (notée REP) du film à 20 °C et à 40 °C, conformément aux protocoles décrits précédemment.

On a obtenu les résultats suivants :

| **Exemple** | **3** | **4** |
|---|---|---|
| **Polymère semi-cristallin** | **exemple 1** | **exemple 2** |
| **RES 20 °C** (en minute) | 40 | 100 |
| **RES 40 °C** (en minute) | 3 | 15 |
| **REP 20 °C** (en %) | 30,8 | 20,4 |
| **REP 40 °C** (en %) | 54,5 | 32,5 |

On constate que pour chaque composition le film de maquillage est bien moins résistant en présence d'eau à 40 °C (eau chaude) qu'en présence d'eau à température ambiante (eau froide). De plus, le film reprend plus d'eau à la température de 40 °C qu'à 20 °C. Le maquillage est donc résistant à l'eau froide et se démaquille facilement à l'eau chaude.

Chaque composition a également été appliquée sur les cils : le maquillage obtenu se démaquille facilement à l'eau chaude (40 °C) sous forme de gaine.

### Exemple 5 :

On a préparé un mascara ayant la composition suivante :

| | | |
|---|---|---|
| - Polyuréthane en dispersion aqueuse vendu sous la dénomination AVALURE UR 425 par la société GOODRICH à 49 % en poids de matières actives | | 18 g MA |
| - Polymère semi-cristallin de l'exemple 2 | | 15 g MA |
| - Hydroxyéthylcellulose | | 1.9 g |
| - Oxyde de fer noir | | 5 g |
| - Propylène glycol | | 5 g |
| - Conservateurs | qs | |
| - Eau | qsp | 100 g |

cette composition forme un film ayant une résistance (notée RES) à l'eau froide (20 °C) égale à 80 minutes et une résistance à l'eau chaude (40 °C) égale à 12 minutes, mesurées conformément au protocole décrit précédemment.
On constate que le film de maquillage obtenu est bien moins résistant en présence d'eau à 40 °C (eau chaude) qu'en présence d'eau à température ambiante (eau froide). Le maquillage est donc résistant à l'eau froide et plus facilement démaquillable à l'eau chaude.

Chaque composition a également été appliquée sur les cils : le maquillage obtenu se démaquille facilement à l'eau chaude (40 °C) sous forme de gaine.

## Revendications

1. Composition comprenant, dans un milieu physiologiquement acceptable, un premier polymère semi-cristallin ayant un point de fusion supérieur ou égal à 30 °C, ledit premier polymère semi-cristallin étant choisi parmi les copolymères séquencés comportant au moins une séquence cristallisable et au moins une séquence amorphe, les homopolymères et les copolymères portant au moins une chaîne latérale cristallisable par motif de répétition, et leurs mélanges, ledit premier polymère semi-cristallin étant choisi parmi les homopolymères d'alkyl(méth)acrylate ou d'alkyl(méth)acrylamide avec un groupe alkyle en C₁₄ à C₂₄, les copolymères de ces monomères avec un monomère hydrophile, et un deuxième polymère filmogène apte à former un film hydrophobe à température ambiante, le polymère semi-cristallin est présent en une teneur allant de 1 à 30 % en poids, par rapport au poids total de la composition et le deuxième polymère filmogène est présent en une teneur en matière sèche allant de 5 % à 60 % en poids, par rapport au poids total de la composition, le deuxième polymère filmogène est un polyuréthane sous forme de particules en dispersion aqueuse.

2. Composition selon la revendication 1, **caractérisée par le fait que** le polymère semi-cristallin a un point de fusion allant de 30 °C à 80 °C, de préférence allant de 30 °C à 60 °C.

3. Composition selon l'une des revendications précédentes, dans laquelle le polymère semi-cristallin présente une masse moléculaire moyenne en nombre supérieur ou égale à 1 000.

4. Composition selon l'une des revendications précédentes, **caractérisée en ce que** le polymère semi-cristallin a une masse moléculaire moyenne en nombre allant de 3 000 à 500 000 et mieux de 4 000 à 150 000.

5. Composition selon l'une des revendications précédentes, **caractérisée en ce que** la séquence cristallisable est de nature différente de la séquence amorphe.

6. Composition selon l'une des revendications précédentes, **caractérisée** en ce le polymère semi-cristallin est choisi parmi les homopolymères et copolymères comportant de 50 à 100 % en poids de motifs résultant de la polymérisation de un ou plusieurs monomères porteurs de de chaîne(s) latérale(s) hydrophobe(s) cristallisable(s).

7. Composition selon l'une quelconque des revendications précédentes, **caractérisée** en par le fait que les polymères semi-cristallins sont des copolymères d'alkyl(méth)acrylate ou d'alkyl (méth)acrylamide avec un groupe alkyle en C₁₄ à C₂₄ avec un monomère hydrophile de nature différente de l'acide (méth)acrylique comme la N-vinylpyrolidone ou l'hydroxyéthyl (méth)acrylate, et leurs mélanges.

8. Composition selon l'une quelconque des revendications précédentes, **caractérisée en ce que** le polymère semi-cristallin est issu d'un monomère à chaîne cristallisable choisi parmi les (méth)acrylates d'alkyle saturés en C₁₄ à C₂₂.

9. Composition selon l'une quelconque des revendications précédentes, **caractérisée en ce que** le polymère semi-cristallin a une chaîne organique cristallisable et/ou une séquence cristallisable représentant au moins 30 % du poids total du polymère.

10. Composition selon l'une quelconque des revendications précédentes, **caractérisée par le fait que** le deuxième polymère filmogène est présent en une teneur allant de 10 % à 45 % en poids, par rapport au poids total de la composition, et de préférence de 15 % à 35 % en poids.

11. Composition selon l'une quelconque des revendications précédentes, **caractérisée par le fait que** le premier polymère semi-cristallin et le deuxième polymère filmogène sont présents dans la composition selon un rapport pondéral deuxième polymère filmogène / premier polymère semi-cristallin allant de 90/10 à 10/90, de préférence de 70/30 à 30/70, et mieux de 60/40 à 40/60.

12. Composition selon l'une quelconque des revendications précédentes, **caractérisée par le fait qu'**elle comprend, en outre, au moins un additif choisi dans le groupe formé par les épaississants, les matières colorantes, les conservateurs, les parfums, les filtres solaires, les agents antiradicaux-libres, les cires, les huiles, les agents hydratants, les vitamines, les protéines, les plastifiants, les sequestrants, les céramides, les agents alcalinisants ou acidifiants, les émollients.

13. Composition selon l'une quelconque des revendications précédentes, **caractérisée par le fait qu'**elle se présente sous la forme de mascara, d'eye-liner, de produit pour les lèvres, de fard à joues ou à paupières, de fond de teint, de produit de maquillage du corps, de produit anti-cernes, de produit pour les ongles, de composition de protection solaire, de coloration de la peau, de produit de soin de la peau.

14. Procédé cosmétique de maquillage ou de soin des matières kératiniques comprenant l'application sur les matières kératiniques d'une composition selon l'une quelconque des revendications 1 à 13.

15. Utilisation d'une composition selon l'une des revendications 1 à 14 pour obtenir un film déposé sur les matières kératiniques résistant à l'eau froide et/ou démaquillable à l'eau chaude.

16. Procédé cosmétique de démaquillage des matières kératiniques maquillées avec une composition selon l'une quelconque des revendications 1 à 14, comprenant au moins une étape de rinçage avec de l'eau chaude portée à une température allant de 35 °C à 50 °C desdites matières kératiniques maquillées.

17. Procédé selon la revendication 16, **caractérisé par le fait que** l'eau chaude ne contient pas d'agent détergent.

## Patentansprüche

1. Zusammensetzung, die in einem physiologisch unbedenklichen Medium ein erstes teilkristallines Polymer mit einem Schmelzpunkt größer oder gleich 30 °C, wobei das erste teilkristalline Polymer aus Blockcopolymeren mit mindestens einem kristallinen Block und mindestens einem amorphen Block, Homopolymeren und Copolymeren mit mindestens einer kristallisierbaren Seitenkette pro Wiederholungseinheit und Mischungen davon ausgewählt ist, wobei das erste teilkristalline Polymer aus Homopolymeren von Alkyl(meth)acrylat oder Alkyl-(meth)acrylamid mit einer C₁₄- bis C₂₄-Alkylgruppe und Copolymeren dieser Monomere mit einem hydrophilen Monomer ausgewählt ist, und ein zweites filmbildendes Polymer, das zur Bildung eines hydrophoben Films bei Umgebungstemperatur befähigt ist, umfasst, wobei das teilkristalline Polymer in einem Gehalt im Bereich von 1 bis 30 Gew.-%, bezogen auf das Gesamtgewicht der Zusammensetzung, vorliegt und das zweite filmbildende Polymer in einem Feststoffgehalt von 5 bis 60 Gew.-%, bezogen auf das Gesamtgewicht der Zusammensetzung, vorliegt und es sich bei dem zweiten filmbildenden Polymer um ein Polyurethan in Form von Teilchen in wässriger Dispersion handelt.

2. Zusammensetzung nach Anspruch 1, **dadurch gekennzeichnet, dass** das teilkristalline Polymer einen Schmelzpunkt im Bereich von 30 °C bis 80 °C, vorzugsweise im Bereich von 30 °C bis 60 °C, aufweist.

3. Zusammensetzung nach einem der vorhergehenden Ansprüche, wobei das teilkristalline Polymer eine zahlenmittlere Molmasse größer oder gleich 1000 aufweist.

4. Zusammensetzung nach einem der vorhergehenden Ansprüche, **dadurch gekennzeichnet, dass** das teilkristalline Polymer eine zahlenmittlere Molmasse im Bereich von 3000 bis 500.000 und noch besser von 4000 bis 150.000 aufweist.

5. Zusammensetzung nach einem der vorhergehenden Ansprüche, **dadurch gekennzeichnet, dass** der kristallisierbare Block anders beschaffen ist als der amorphe Block.

6. Zusammensetzung nach einem der vorhergehenden Ansprüche, **dadurch gekennzeichnet, dass** das teilkristalline Polymer aus Homopolymeren und Copolymeren mit 50 bis 100 Gew.-% Wiederholungseinheiten, die sich aus der Polymerisation von einem oder mehreren Monomeren mit einer oder mehreren kristallisierbaren hydrophoben Seitenketten ergeben, ausgewählt ist.

7. Zusammensetzung nach einem der vorhergehenden Ansprüche, **dadurch gekennzeichnet, dass** es sich bei den teilkristallinen Polymeren um Copolymere von Alkyl(meth)acrylat oder Alkyl(meth)acrylamid mit einer C₁₄- bis C₂₄-Alkylgruppe mit einem hydrophilen Monomer, das anders beschaffen ist als (Meth)acrylsäure, wie N-Vinylpyrrolidon oder Hydroxyethyl(meth)acrylat, oder Mischungen davon handelt.

8. Zusammensetzung nach einem der vorhergehenden Ansprüche, **dadurch gekennzeichnet, dass** sich das teilkristalline Polymer von einem Monomer mit kristallisierbarer Kette, das aus gesättigten C₁₄-bis C₂₂-Alkyl(meth)acrylaten ausgewählt ist, ableitet.

9. Zusammensetzung nach einem der vorhergehenden Ansprüche, **dadurch gekennzeichnet, dass** das teilkristalline Polymer eine kristallisierbare organische Kette und/oder einen kristallisierbaren Block, die bzw. der mindestens 30 % des Gesamtgewichts des Polymers ausmachen, aufweist.

10. Zusammensetzung nach einem der vorhergehenden Ansprüche, **dadurch gekennzeichnet, dass** das zweite filmbildende Polymer in einem Gehalt im Bereich von 10 bis 45 Gew.-%, bezogen auf das Gesamtgewicht der Zusammensetzung, und vorzugsweise 15 bis 35 Gew.-%, vorliegt.

11. Zusammensetzung nach einem der vorhergehenden Ansprüche, **dadurch gekennzeichnet, dass** das erste teilkristalline Polymer und das zweite filmbildende Polymer in der Zusammensetzung gemäß einem Gewichtsverhältnis von filmbildendem Polymer zu erstem teilkristallinem Polymer von 90/10 bis 10/90, vorzugsweise 70/30 bis 30/70 und noch besser 60/40 bis 40/60 vorliegen.

12. Zusammensetzung nach einem der vorhergehenden Ansprüche, **dadurch gekennzeichnet, dass** sie außerdem mindestens ein Additiv aus der Gruppe bestehend aus Verdickern, Farbstoffen, Konservierungsstoffen, Duftstoffen, Sonnenschutzfiltern, Radikalfängern, Wachsen, Ölen, Feuchtigkeitsspendern, Vitaminen, Proteinen, Weichmachern, Sequestriermitteln, Ceramiden, Alkalinisierungs- oder Ansäuerungsmitteln und Emollientien umfasst.

13. Zusammensetzung nach einem der vorhergehenden Ansprüche, **dadurch gekennzeichnet, dass** sie in Form eines Mascaras, eines Eyeliners, eines Produkts für die Lippen, eines Wangenrouges oder eines Lidschattens, einer Make-up-Grundlage, eines Körperschminkprodukts, eines Produkts gegen Falten, eines Produkts für die Nägel, eines Sonnenschutzmittels, eines Hautfärbemittels oder eines Hautpflegeprodukts vorliegt.

14. Kosmetisches Verfahren zum Schminken oder Pflegen von Keratinmaterialien, bei dem man auf die Keratinmaterialien eine Zusammensetzung nach einem der Ansprüche 1 bis 13 aufbringt.

15. Verwendung einer Zusammensetzung nach einem der Ansprüche 1 bis 14 zum Erhalt eines auf Keratinfasern aufgebrachten Films, der gegenüber kaltem Wasser beständig ist und/oder mit warmem Wasser entfernt werden kann.

16. Kosmetisches Verfahren zum Abschminken von geschminkten Keratinmaterialien mit einer Zusammensetzung nach einem der Ansprüche 1 bis 14, das mindestens einen Schritt des Spülens der geschminkten Keratinmaterialien mit auf eine Temperatur im Bereich von 35 °C bis 50 °C gebrachtem Wasser umfasst.

17. Verfahren nach Anspruch 16, **dadurch gekennzeichnet, dass** das warme Wasser kein Reinigungsmittel enthält.

## Claims

1. Composition comprising, in a physiologically acceptable medium, a first semi-crystalline polymer with a melting point of greater than or equal to 30°C, said first semi-crystalline polymer being chosen from block copolymers comprising at least one crystallizable block and at least one amorphous block, homopolymers and copolymers bearing at least one crystallizable side chain per repeating unit, and mixtures thereof, said first semi-crystalline polymer being chosen from alkyl (meth)acrylate or alkyl(meth)acrylamide homopolymers with a C₁₄ to C₂₄ alkyl group, and copolymers of these monomers with a hydrophilic monomer, the semi-crystalline polymer is present in a content ranging from 0.1% to 30% by weight, relative to the total weight of the composition, and the second film-forming polymer is present in a solid content ranging from 5% to 60% by weight, relative to the total weight of the composition, the second film-forming polymer is a polyurethane in the form of particles in aqueous dispersion.

2. Composition according to Claim 1, **characterized in that** the semi-crystalline polymer has a melting point ranging from 30°C to 80°C and preferably ranging from 30°C to 60°C.

3. Composition according to either of the preceding claims, in which the semi-crystalline polymer has a number-average molecular mass of greater than or equal to 1 000.

4. Composition according to one of the preceding claims, **characterized in that** the semi-crystalline polymer has a number-average molecular mass ranging from 3 000 to 500 000 and better still from 4 000 to 150 000.

5. Composition according to one of the preceding claims, **characterized in that** the crystallizable block is of different nature from the amorphous block.

6. Composition according to one of the preceding claims, **characterized in that** the semi-crystalline polymer is chosen from homopolymers and copolymers comprising from 50% to 100% by weight of units resulting from the polymerization of one or more monomers bearing crystallizable hydrophobic side chain(s).

7. Composition according to any one of the preceding claims, **characterized in that** the semi-crystalline polymers are copolymers of alkyl (meth)acrylate or of alkyl(meth)acrylamide with a C₁₄ to C₂₄ alkyl group, with a hydrophilic monomer of different nature from (meth)acrylic acid, for instance N-vinylpyrrolidone or hydroxyethyl (meth)acrylate, and mixtures thereof.

8. Composition according to any one of the preceding claims, **characterized in that** the semi-crystalline polymer is derived from a monomer containing a crystallizable chain chosen from saturated C₁₄ to C₂₂ alkyl (meth)acrylates.

9. Composition according to any one of the preceding claims, **characterized in that** the semi-crystalline polymer has a crystallizable organic chain and/or a crystallizable block representing at least 30% of the total weight of the polymer.

10. Composition according to any one of the preceding claims, **characterized in that** the second film-forming polymer is present in a content ranging from 10% to 45% by weight and preferably from 15% to 35% by weight relative to the total weight of the composition.

11. Composition according to any one of the preceding claims, **characterized in that** the first semi-crystalline polymer and the second film-forming polymer are present in the composition in a second film-forming polymer/first semi-crystalline polymer weight ratio ranging from 90/10 to 10/90, preferably from 70/30 to 30/70 and better still from 60/40 to 40/60.

12. Composition according to any one of the preceding claims, **characterized in that** it also comprises at least one additive chosen from the group formed by thickeners, dyestuffs, preserving agents, fragrances, sunscreens, free-radical scavengers, waxes, oils, moisturizers, vitamins, proteins, plasticizers, sequestrants, ceramides, acidifying or basifying agents, and emollients.

13. Composition according to any one of the preceding claims, **characterized in that** it is in the form of a mascara, an eyeliner, a product for the lips, a face powder, an eyeshadow, a foundation, a makeup product for the body, a concealer product, a product for the nails, an antisun composition, a skin colouring composition or a skincare product.

14. Cosmetic makeup or care process for keratin materials, comprising the application to the keratin materials of a composition according to any one of Claims 1 to 13.

15. Use of a composition according to one of Claims 1 to 14, to obtain a film applied to keratin materials that is resistant to cold water and/or that can be removed with warm water.

16. Cosmetic process for removing makeup from keratin materials made up with a composition according to any one of Claims 1 to 14, comprising at least one step of rinsing with warm water brought to a temperature ranging from 35°C to 50°C, of the said made-up keratin materials.

17. Process according to Claim 16, **characterized in that** the warm water contains no detergent.
